# EUROPEAN PATENT APPLICATION

(11) **EP 3 581 112 A1**
(43) Date of publication of application: **18.12.2019**
(21) Application number: 18750754.6
(22) Date of filing: 07.02.2018
(51) Int. Cl.: A61B 8/12, A61B 5/04

(54) **MEDICAL DEVICE, MEDICAL SYSTEM, METHOD FOR INSERTING MEDICAL DEVICE, AND OBSERVATION METHOD USING MEDICAL DEVICE**

(30) Priority: 08.02.2017 JP 2017021655
(71) Applicant: Terumo Kabushiki Kaisha, Tokyo 151-0072 (JP)
(72) Inventor: SHIMIZU Katsuhiko, Ashigarakami-gun Kanagawa 259-0151 (JP); OOKUBO Itaru, Ashigarakami-gun Kanagawa 2590151 (JP); SAKAMOTO Yasukazu, Ashigarakami-gun Kanagawa 259-0151 (JP); HOSONO Hiroki, Ashigarakami-gun Kanagawa 259-0151 (JP)
(74) Representative: KIPA AB
(86) International application number: PCT/JP2018/004262
(87) International publication number: WO 2018/147341

(57) **Abstract**

Provided is a medical device including: a first elongated body; an electrode that is fixed to the annular expansion portion of the first elongated body; a second elongated body that has a distal portion and a proximal portion, a first lumen communicating from the distal portion to the proximal portion, and a second lumen parallel to the first lumen at the distal portion; and an ultrasound element that is configured to move inside the first lumen of the second elongated body in an axial direction, in which the first elongated body is inserted into the second lumen, and the linear portion of the first elongated body is positioned in a direction orthogonal to the axial direction of the ultrasound element, and the ultrasound element receives ultrasound reflected from the first elongated body and ultrasound reflected from a body-cavity around the second elongated body.

## Description

### Technical Field

The present disclosure relates to a medical device, a medical system, a method for inserting the medical device, and an observation method using the medical device.

### Background Art

In the related art, a treatment for disconnecting transmission of electrical signals between specific sites on an inner body lumen wall is known. For example, in order to treat atrial fibrillation, a so-called ablation treatment is performed in which an abnormal excitation from a pulmonary vein is electrically disconnected so as not to be transmitted to a left atrium by cauterizing a pulmonary vein ostium or the like, which is a junction of the pulmonary vein and the left atrium. In a case of such treatment for disconnecting the transmission of the electrical signals between the specific sites on the inner body lumen wall, it is important to check a success of the treatment.

PTL 1 discloses a medical device provided with an electrode array which is shape-memorized in a ring shape and in which a plurality of electrodes are arranged. According to the medical device disclosed in PTL 1, by contacting the electrode array with an inner wall of a body lumen, it is possible to measure an electrical property such as an impedance of a contact site, which can be used to determine whether or not the contact site is electrically disconnected.

PTL 2 discloses a monitoring device capable of obtaining information about cauterization such as a cauterization depth inside a body tissue by transmitting an ultrasound pulse to the body tissue. According to the monitoring device disclosed in PTL 2, since the information about the cauterization can be obtained, the cauterization at each point can be sufficiently performed.

### Citation List

### Patent Literature

PTL 1: US Patent No. 9168080
PTL 2: Japanese Patent No. 5829526

### Summary of Invention

### Technical Problem

However, in the medical device disclosed in PTL 1, when the contact site is not electrically disconnected, there is a problem that it is difficult to determine at which point it is necessary to perform a procedure such as a cauterization to electrically disconnect the contact portion. Even when the contact site is electrically disconnected, there is a problem that the transmission of the electrical signals may be restored after a certain period of time, because there is a point where the cauterization depth is shallow so that the cauterization is insufficient, for example.

The monitoring device disclosed in PTL 2 has a problem that it is difficult to reliably disconnect transmission of electrical signals between specific sites.

In view of the above problems, it is an object of the present disclosure to provide a medical device, a medical system, a method for inserting the medical device, and an observation method using the medical device, which are capable of easily grasping a site with insufficient cauterization when performing a treatment for disconnecting transmission of electrical signals between specific sites on an inner body lumen wall.

### Solution to Problem

As an aspect of the present invention, there is provided a medical device including: a first elongated body that has a linear portion and an annular expansion portion configured to expand in an annular shape; an electrode that is fixed to the annular expansion portion of the first elongated body and is configured to detect an electrical property of an inner wall of a body lumen; a second elongated body that has a distal portion and a proximal portion, the second elongated body including a first lumen communicating from the distal portion to the proximal portion, and a second lumen parallel to the first lumen at the distal portion; and an ultrasound element that is fixed to a distal portion of a shaft configured to rotate in a circumferential direction, is configured to move inside the first lumen of the second elongated body in an axial direction, and is configured to transmit and receive ultrasound, in which the annular expansion portion is configured to expand in an annular shape which is larger than an outer diameter of the second elongated body, the first elongated body is inserted into the second lumen, and the linear portion of the first elongated body is positioned in a direction orthogonal to the axial direction of the ultrasound element, and the ultrasound element receives ultrasound reflected from the first elongated body and ultrasound reflected from a body-cavity around the second elongated body.

In the medical device as an embodiment of the present invention, a positional relationship of the ultrasound element with respect to the second elongated body in a circumferential direction of the second elongated body, is fixed.

The medical device as the embodiment of the present invention, a plurality of the electrodes are provided, and the plurality of the electrodes include two or more electrodes fixed at different positions along a circumferential direction of the first elongated body.

In the medical device as the embodiment of the present invention, the ultrasound element is disposed closer to a proximal side than the annular expansion portion of the first elongated body.

In the medical device as the embodiment of the present invention, the annular expansion portion is configured to move to the same position as the ultrasound element in the axial direction of the ultrasound element, and the ultrasound element receives ultrasound reflected from the annular expansion portion.

In the medical device as the embodiment of the present invention, a distal end of the second lumen is positioned closer to a distal side than a distal end of the first lumen.

In the medical device as the embodiment of the present invention, the second lumen extends along an extending direction of the first lumen.

In the medical device as the embodiment of the present invention, the first elongated body has a curved portion that is curved so as to be separated radially outward of the ultrasound element with respect to a position of the ultrasound element.

As another aspect of the present invention, there is provided a medical system including: a medical device; and an ultrasound diagnostic apparatus, in which the medical device includes a first elongated body that has a linear portion and an annular expansion portion configured to expand in an annular shape, a plurality of electrodes that are fixed to the annular expansion portion of the first elongated body and is configured to detect an electrical property of an inner wall of a body lumen, a second elongated body that has a distal portion and a proximal portion, a first lumen communicating from the distal portion to the proximal portion, and a second lumen parallel to the first lumen at the distal portion, and an ultrasound element that is fixed to a distal portion of a shaft configured to rotate in a circumferential direction, is configured to move inside the first lumen of the second elongated body in an axial direction, and transmits and receives ultrasound, the annular expansion portion is configured to expand in an annular shape which is larger than an outer diameter of the second elongated body, the first elongated body is inserted into the second lumen, and the linear portion of the first elongated body is positioned in a direction orthogonal to the axial direction of the ultrasound element, and the ultrasound element receives ultrasound reflected from the first elongated body and ultrasound reflected from a body-cavity around the second elongated body, and transmits a signal which is based on the received ultrasound to the ultrasound diagnostic apparatus, the ultrasound diagnostic apparatus includes a display unit and a control unit, in which the control unit causes the display unit to display an image that is configured to specify a position of the electrode in a circumferential direction of the ultrasound element based on the signal from the ultrasound element.

As still another aspect of the present invention, there is provided a method for inserting a medical device that includes an elongated body having an electrode configured to detect an electrical property of an inner wall of a body lumen at a distal portion and an ultrasound element configured to move along an extending direction of the elongated body with respect to the elongated body, the method including: disposing the distal portion of the elongated body on the inner wall of the body lumen; and moving the ultrasound element toward the electrode along the extending direction of the elongated body.

The method for inserting the medical device as the embodiment of the present invention, further includes: checking an electrocardiogram based on the electrical property of the inner wall of the body lumen by the electrode; checking a position of the elongated body based on an image which is based on a signal from the ultrasound element displayed on a display unit of an ultrasound diagnostic apparatus; specifying a position of an electrode corresponding to an insufficiently cauterized position in the inner wall of the body lumen in a circumferential direction of the ultrasound element, based on the position of the elongated body and the electrocardiogram; and delivering a cauterization device to the position of the specified electrode and cauterizing the inner wall of the body lumen.

As still another aspect of the present invention, there is provided an observation method using a medical device that includes an ultrasound element, including: acquiring positional information of surroundings using ultrasound in a first frequency band by the ultrasound element when opening a left atrium from a right atrium; and acquiring positional information of surroundings using ultrasound in a second frequency band, which is a higher frequency band than the first frequency band, by the ultrasound element when performing an ablation treatment.

### Advantageous Effects of Invention

According to the present disclosure, it is possible to provide a medical device, a medical system, a method for inserting the medical device, and an observation method using the medical device, which are sufficiently cauterized at each point and can detect that electrical signals between specific sites are reliably disconnected when performing a treatment for disconnecting transmission of the electrical signals between the specific sites on an inner body lumen wall.

### Brief Description of Drawings

[Fig. 1] Fig. 1 is a perspective view of a medical device according to one embodiment of the present disclosure.
[Fig. 2] Fig. 2 is a cross-sectional view of the medical device shown in Fig.1.
[Fig. 3] Fig. 3 is a front view of the medical device shown in Fig. 1 viewed from a far side.
[Fig. 4] Fig. 4 is a view showing a first modification example of the medical device shown in Fig. 1.
[Fig. 5] Fig. 5 is a view showing a second modification example of the medical device shown in Fig. 1.
[Fig. 6] Fig. 6 is a view showing a third modification example of the medical device shown in Fig. 1.
[Fig. 7] Fig. 7 is a front view of the medical device shown in Fig. 6 viewed from a far side.
[Fig. 8] Fig. 8 is a view showing a fourth modification example of the medical device shown in Fig. 1.
[Fig. 9] Fig. 9 is a schematic view of a medical system according to the embodiment of the present disclosure.
[Fig. 10] Fig. 10 is a view showing a right atrium.
[Fig. 11] Fig. 11 is a view showing a left atrium.
[Fig. 12] Fig. 12 is a flowchart showing each step of a method for inserting the medical device shown in Fig. 1 into a heart and a method for observing the inside of the heart using the medical device shown in Fig. 1.
[Fig. 13] Fig. 13 is a view showing an example of an image generated based on positional information acquired by an ultrasound element.
[Fig. 14] Fig. 14 is a view showing a part including a distal end of a guide wire of the medical device shown in Fig. 1.
[Fig. 15] Fig. 15 is a view showing a state in which the medical device shown in Fig. 6 is inserted into a sheath.

### Description of Embodiments

Hereinafter, each embodiment of the present disclosure will be described with reference to the drawings. The same reference numeral is given to a common member in each drawing. In the present specification, a side of the medical device to be inserted into a body lumen is referred to as "far side" or "distal side", and a side close to hand to operate is referred to as "near side" or "proximal side".

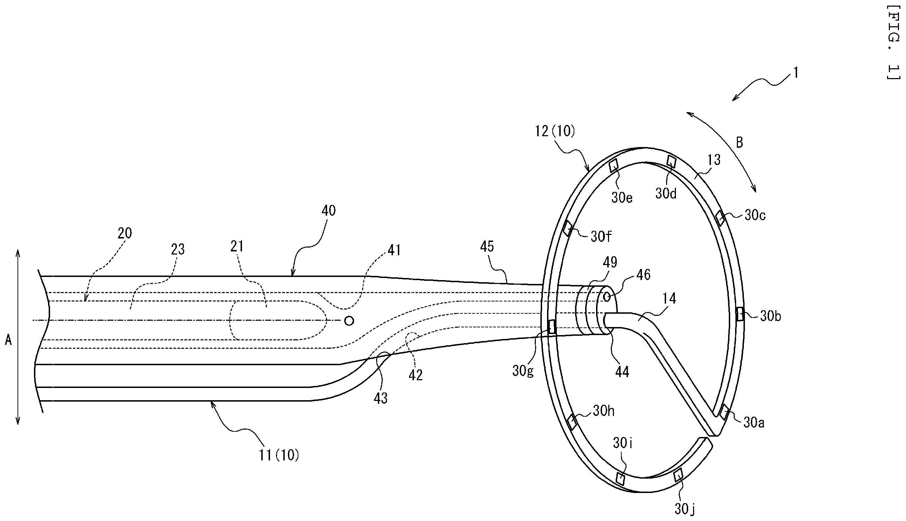

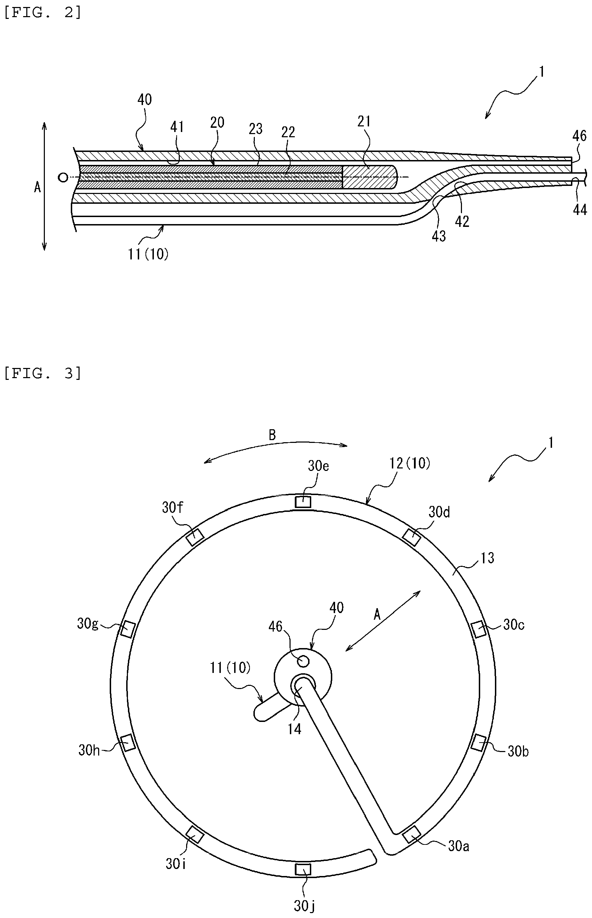

Fig. 1 is a perspective view showing a medical device 1 according to one embodiment of the present disclosure, Fig. 2 is a cross-sectional view of the medical device 1, and Fig. 3 is a front view of the medical device 1 viewed from a far side.

As shown in Figs. 1 to 3, the medical device 1 includes a guide wire 10 as a first elongated body, an ultrasound inspector 20, electrodes 30a to 30j, and a catheter 40 as a second elongated body. The catheter 40 internally defines sections of a first lumen 41 into which the ultrasound inspector 20 can be inserted and a second lumen 42 into which the guide wire 10 can be inserted. Figs. 1 to 3 show a state in which the ultrasound inspector 20 is inserted into the first lumen 41 and the guide wire 10 is inserted into the second lumen 42. Hereinafter, unless otherwise specified, the ultrasound inspector 20 and the guide wire 10 will be described as being inserted into the first lumen 41 and the second lumen 42, respectively.

The guide wire 10 extends from the near side to the far side. The guide wire 10 has a linear portion 11, an annular expansion portion 12 provided at an end portion of the far side, and a first bent portion 14 positioned between the linear portion 11 and the annular expansion portion 12. The annular expansion portion 12 is made of, for example, a metal such as a shape memory alloy, and in an environment where an external force of a certain level or less acts, the annular expansion portion 12 is shape-memorized so as to expand in an annular shape. The shape memory alloy is also called a super elastic alloy, and is an alloy that exhibits elasticity at least at living body temperature (for example, around 37°C) . The super elastic alloy is not particularly limited, and an alloy of nickel and titanium is preferred.

In the states shown in Figs . 1 and 3, the annular expansion portion 12 is positioned closer to a far side than the far end of the second lumen 42 (far side communication hole 44 to be described later), and is expanded in an annular shape. The annular expansion portion 12 expands outward in a radial direction A of the linear portion 11 of the guide wire 10 and extends along a circumferential direction B of the linear portion 11 of the guide wire 10. With an expanded state in an annular shape, the annular expansion portion 12 is larger than an outer diameter of the catheter 40. As shown in Fig. 3, when viewed from the distal side of the guide wire 10, the linear portion 11 of the guide wire 10 and the catheter 40 are positioned inside the annular expansion portion 12 expanded in an annular shape which is larger than the outer diameter of the catheter 40. In a state of being inserted into the second lumen 42, the annular expansion portion 12 deforms in a linear shape same as the linear portion 11. Hereinafter, even in the state where the annular expansion portion 12 is expanded in an annular shape, the radial direction of the linear portion 11 of the guide wire 10 is simply described as "radial direction A", and the circumferential direction of the linear portion 11 of the guide wire 10 is simply described as "circumferential direction B". Hereinafter, unless otherwise specified, the annular expansion portion 12 will be described as being in an expanded state in an annular shape.

The electrodes 30a to 30j are fixed to the annular expansion portion 12, and each of the electrodes 30a to 30j is fixed at different positions along the annular expansion portion 12 in an extending direction, that is, along the guide wire 10 in the circumferential direction B. As shown in Fig. 3, when viewed from the far side, the electrodes 30a to 30j are arranged counterclockwise at substantially equal intervals in this order. Hereinafter, when the electrodes 30a to 30j are not distinguished, the electrodes are collectively referred to as the electrodes 30.

The electrodes 30 can detect an electrical property of an inner wall of a body lumen by contacting the inner wall of the body lumen. As the electrical property, for example, a potential difference or the like between the electrodes 30 and other electrodes which are in contact with another site of the living body can be used. The electrodes 30 are arranged so as to be exposed from the far end 13 of the annular expansion portion 12, and by pressing the far end 13 of the annular expansion portion 12 against the inner wall of the body lumen, the electrodes 30 can be in contact with the inner wall of the body lumen.

The ultrasound inspector 20 includes an ultrasound element 21, a shaft 22 and a tube 23.

As shown in Figs. 1 and 2, the ultrasound element 21 is provided at the far end of the ultrasound inspector 20. The ultrasound element 21 may be provided at a position other than the far end of the ultrasound inspector 20. A positional relationship of the ultrasound element 21 in the circumferential direction B of the guide wire 10 may be fixed with respect to the guide wire 10. As described later, this is realized by the catheter 40 constituting a rotation restriction member that restricts a rotation of the guide wire 10 by a shape of the second lumen 42, for example. In this way, by fixing the positional relationship of the ultrasound element 21 in the circumferential direction B of the guide wire 10, unintended changes in the positional relationship can be suppressed. Therefore, the positional relationship of the electrodes 30a to 30j in the circumferential direction with respect to the ultrasound element 21 can be grasped more reliably, so that a position where cauterization is insufficient at the time of ablation treatment described later can be grasped more reliably.

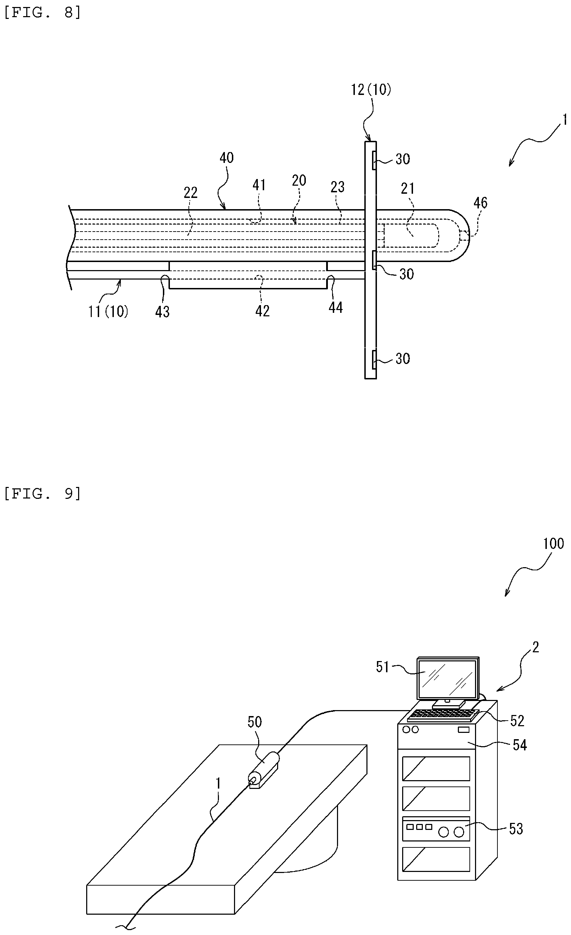

As shown in Figs. 1 and 2, a central axis O of the ultrasound element 21 is along the extending direction of the linear portion 11 of the guide wire 10. The ultrasound element 21 acquires positional information around the central axis O by transmitting and receiving ultrasound along the radial direction. Specifically, the ultrasound element 21 includes at least an ultrasound transmitter and an ultrasound receiver, transmits ultrasound along the radial direction by the ultrasound transmitter while rotating around the central axis O, and acquires signals about surrounding positional information by receiving reflected ultrasound by the ultrasound receiver and performing signal processing. The signal processing may be performed by the ultrasound element 21 or may be performed by an ultrasound diagnostic apparatus 2 (see Fig. 9) connected to the ultrasound element 21. In a state of being inserted into the body lumen, the ultrasound element 21 can acquire signals including cross-sectional information from the inner wall of the body lumen by receiving ultrasound reflected from the body lumen. The ultrasound element 21 preferably transmits ultrasound at a predetermined angle, for example, 3° to 18°, with respect to the radial direction. In this way, by transmitting the ultrasound at the predetermined angle with respect to the radial direction, it is possible to suppress a detection (ring-down) of the ultrasound reflected from an inner circumferential surface of the first lumen 41.

By receiving the ultrasound reflected from the linear portion 11 of the guide wire 10, the ultrasound element 21 can acquire the positional information of the guide wire 10 in the circumferential direction around the central axis O of the ultrasound element 21. If positional information of the guide wire 10 in the circumferential direction B can be acquired and unless the positional relationship in the circumferential direction B with respect to the guide wire 10 is changed, the ultrasound element 21 can indirectly acquire positional information of each of the electrodes 30a to 30j fixed to the annular expansion portion 12 of the guide wire 10 in the circumferential direction B.

The ultrasound element 21 can acquire surrounding positional information using ultrasound in a first frequency band, and can also acquire surrounding positional information using ultrasound in a second frequency band which is a higher frequency band than the first frequency band. As a result, it is possible to obtain a relatively wide range of positional information in the body lumen by the ultrasound in the first frequency band, and it is also possible to obtain a relatively narrow range of positional information in the body lumen by the ultrasound in the second frequency band with higher accuracy. The ultrasound element 21 may be configured with a single element capable of transmitting the ultrasound in the first frequency band and the second frequency band, or may be configured by using an element capable of transmitting the ultrasound in the first frequency band and an element capable of transmitting the ultrasound in the second frequency band in combination. The first frequency band is, for example, 6 to 12 MHz, and is useful when opening a left atrium from a right atrium of a heart by penetrating with a needle such as a Brockenbrough needle by using a Brockenbrough method, for example. The second frequency band is, for example, 15 to 20 MHz, and is useful for observing a myocardial tissue state when performing an ablation treatment, for example.

As shown in Fig. 2, the shaft 22 fixes the ultrasound element 21 on the near side along the central axis O of the ultrasound element 21. The shaft 22 is a flexible linear member extending along the guide wire 10 in an extending direction, and is rotatable in the circumferential direction about the central axis O of the ultrasound element 21. The ultrasound element 21 acquires positional information around the central axis O by rotating around the central axis O in interlock with the rotation of the shaft 22 in the circumferential direction. The outer diameter of the shaft 22 is smaller than the outer diameter of the ultrasound element 21.

As shown in Fig. 2, the tube 23 is a flexible tubular member that covers the shaft 22 in the circumferential direction. The tube 23 can slide in the extending direction with respect to the catheter 40 without inhibiting the rotation of the shaft 22. A proximal portion of the tube 23 is harder than a distal portion of the tube 23 in order to easily transmit a hand-pushing force on the proximal side of the medical device 1 to the distal side of the medical device 1. The proximal portion of the tube 23 is double-layered or doubled. The ultrasound element 21 tends to have an outer diameter larger due to transmit the ultrasound particularly in a low frequency band such as the first frequency band. For example, the size of the ultrasound element 21 is 0.6 mm to 3.0 mm in length, 0.6 mm to 3.0 mm in width, and 50 µm to 350 µm in thickness. In this case, if the shaft 22 having an outer diameter smaller than the size of the ultrasound element 21 is used, the rotational stability is degraded. Therefore, by covering the shaft 22 in the circumferential direction with the tube 23, the rotational stability of the shaft 22 can be improved.

As a material of the tube 23, in order to reduce a frictional resistance with the shaft 22, a polyolefin or the like is preferable.

As shown in Figs. 1 and 2, the catheter 40 has the distal portion 45 which is an end portion of the far side and the proximal portion (not shown) which is an end portion of the near side, and is a rapid exchange (RX) type catheter in which the second lumen 42 is defined only at the distal portion 45. The first lumen 41 of the catheter 40 communicates from the distal portion 45 of the catheter 40 to the proximal portion (not shown). In other words, the first lumen 41 communicates with the outside through an opening (not shown) provided at the proximal portion, and communicates with the outside through an opening 46 provided at the distal portion 45 to enable priming. An inner diameter of the opening 46 is preferably smaller than the outer diameter of the ultrasound element 21. As a result, the ultrasound element 21 inserted into the first lumen 41 is suppressed from being discharged to the outside through the opening 46, so a position of the ultrasound element 21 is restricted closer to a proximal side than the annular expansion portion 12 of the guide wire 10. The ultrasound element 21 is movable with respect to the guide wire 10 in the extending direction of the guide wire 10. Since the first lumen 41 is defined inward in the radial direction A of the guide wire 10 than the annular expansion portion 12 of the guide wire 10, the ultrasound element 21 is disposed inward in the radial direction A of the guide wire 10 than the annular expansion portion 12.

As shown in Fig. 2, the second lumen 42 of the catheter 40 is positioned at a position different from the first lumen 41 at the distal portion 45. In other words, the second lumen 42 is positioned in a direction orthogonal to the extending direction of the first lumen 41 at the distal portion 45. The second lumen 42 is parallel to the first lumen 41. In other words, the second lumen 42 extends along the extending direction of the first lumen 41. In the present example, the second lumen 42 extends from a near side communication hole 43 formed on the side of the catheter 40 to a far side communication hole 44 formed on the far end of the catheter 40. By setting the first lumen 41 and the second lumen 42 in the above-described positional relationship, the ultrasound element 21 can be disposed along the extending direction of the linear portion 11 which is closer to a proximal side than the annular expansion portion 12 of the guide wire 10. The guide wire 10 is inserted in a state where the second lumen 42 is penetrated, and can be freely moved with respect to the catheter 40 in the extending direction. At this time, the linear portion 11 of the guide wire 10 is positioned in a direction orthogonal to an axial direction of the ultrasound element 21. The inner diameter of the second lumen 42 is smaller than the inner diameter of the first lumen 41. The annular expansion portion 12 can be accommodated in the second lumen 42 by being deformed linearly along the inner wall of the second lumen 42.

The catheter 40 may be configured with a rotation restriction member that restricts the rotation of the guide wire 10 according to the shape of the second lumen 42. Specifically, the shape of the cross section orthogonal to the extending direction of the second lumen 42 may have a shape for preventing the rotation of the guide wire 10. For example, it is assumed that the cross section orthogonal to the extending direction of the second lumen 42 is an elliptical shape having an inner diameter shorter than a long diameter of the cross section along the extending direction of the guide wire 10 in part. Thereby, the ultrasound element 21 has a fixed positional relationship in the circumferential direction B with respect to the guide wire 10. The shape of the cross section orthogonal to the extending direction of the second lumen 42 may be any shape as long as the shape prevents the rotation of the guide wire 10, and various shapes can be adopted without being limited to the above-described elliptical shape.

As described above, the second lumen 42 extends from the near side communication hole 43 formed on the side of the catheter 40 to the far side communication hole 44 formed on the far end of the catheter 40. The central axis (which coincides with the central axis O of the ultrasound element 21 in Fig. 2) of the first lumen 41 is in a positional relationship penetrating the center of the far side communication hole 44 of the second lumen 42. With such a positional relationship, a position of the ultrasound element 21 can be made to substantially coincide with a center position of the annular expansion portion 12 in the expanded state in an annular shape, in the front view in Fig. 3.

When a radial direction distance of the guide wire 10 from the central axis O of the ultrasound element 21 is extremely close, since the guide wire 10 becomes an obstacle, a detection of surrounding positional information may be inhibited. Therefore, the guide wire 10 is preferably separated radially outward from the ultrasound element 21 at the position of the ultrasound element 21 in the extending direction of the catheter 40 and at the position radially outward of the ultrasound element 21. In other words, the guide wire 10 is preferably shaped so as to be separated radially outward of the ultrasound element 21 with respect to the position of the ultrasound element 21. In the present embodiment, since the near side communication hole 43 is formed on the side of the catheter 40, the part of the catheter 40 extending outward from the near side communication hole 43 is easily separated from the side of the catheter 40. Therefore, by disposing the ultrasound element 21 in the vicinity of the proximal side of the near side communication hole 43, it is possible to easily secure the radial direction distance between the ultrasound element 21 and the guide wire 10. As a result, the guide wire 10 can be prevented from becoming an obstacle to acquire the positional information by the ultrasound element 21.

The catheter 40 may be switchable between a fixed state in which the ultrasound inspector 20 is fixed inside the first lumen 41 and a released state in which the ultrasound inspector 20 is released from the first lumen 41. In this way, for example, if the catheter 40 is in the fixed state when it is inserted into the body lumen, even if the catheter 40 alone has low rigidity and insertion is difficult, since the ultrasound inspector 20 can be inserted into the body lumen simultaneously with the catheter 40, the rigidity can be increased. For example, if the catheter 40 is in the fixed state when acquiring the surrounding positional information using the ultrasound element 21, an unintended movement of the ultrasound element 21 along the extending direction of the catheter 40 can be suppressed.

As shown in Fig. 1, a contrast marker 49 made of an X-ray non-transmittable material may be appropriately disposed, for example, in the vicinity of the distal end (far end) of the catheter 40. Thereby, the position of the catheter 40 can be checked by an X-ray photography. Examples of the X-ray non-transmittable material include platinum, gold, silver, titanium, tungsten and the like.

The catheter 40 is not limited to the RX type catheter, and may be another shape catheter, for example, an over-the-wire (OTW) type catheter. However, the RX type catheter is preferred in that it is easy to configure as described above.

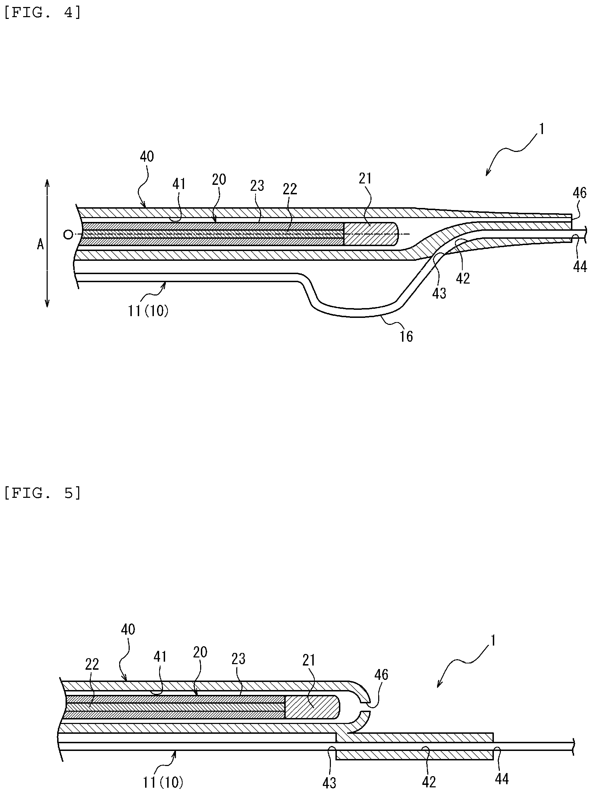

Fig. 4 is a view showing a first modification example of the medical device 1. As shown in Fig. 4, the guide wire 10 may have a curved portion 16 that is curved so as to be separated radially outward of the ultrasound element 21 with respect to the position of the ultrasound element 21. With this configuration, the guide wire 10 can be further prevented from becoming an obstacle to acquire the positional information by the ultrasound element 21. The curved portion 16 is preferably separated from the ultrasound element 21 by 1 mm to 10 mm, and more preferably 3 mm to 5 mm.

Fig. 5 is a view showing a second modification example of the medical device 1. As shown in Fig. 5, the distal end of the far side of the second lumen 42 may be positioned closer to a distal side than the distal end of the far side of the first lumen 41. In other words, the far side communication hole 44 of the second lumen 42 may be positioned closer to a distal side than the opening 46 of the first lumen 41. As described above, the inner diameter of the opening 46 may be smaller than the outer diameter of the ultrasound element 21.

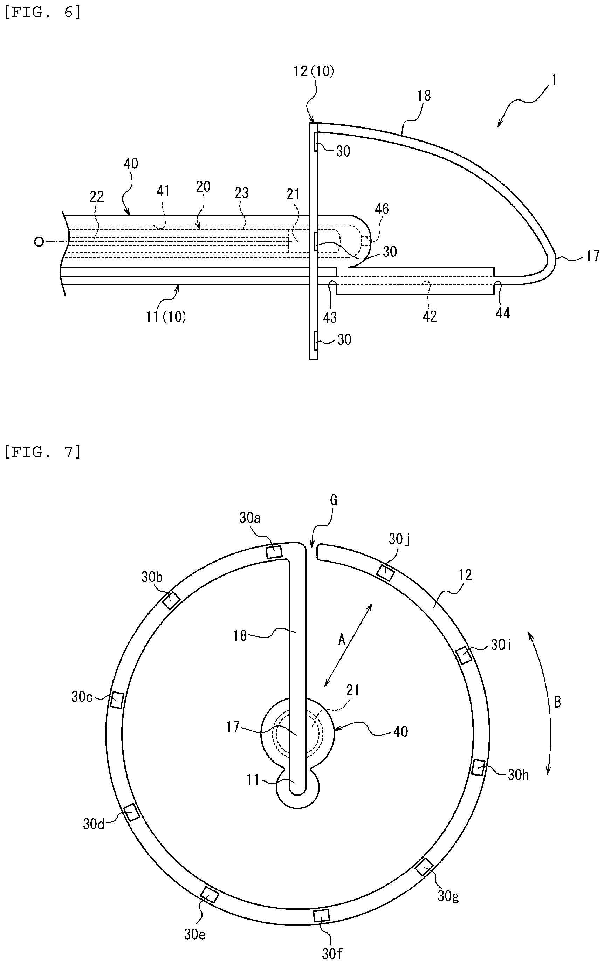

Fig. 6 is a view showing a third modification example of the medical device 1. Fig. 7 is a front view of the medical device 1 according to the third modification example viewed from the far side. As shown in Fig. 6, the linear portion 11 of the guide wire 10 may be bendable via a second bent portion 17 which is closer to a distal side than the second lumen 42. The annular expansion portion 12 is not restricted in position on the distal side of the ultrasound element 21 by turning-back the annular expansion portion 12 to the proximal side via the second bent portion 17 of the linear portion 11. Thereby, the annular expansion portion 12 can move to the same position as the ultrasound element 21 in the axial direction of the ultrasound element 21, and can further move closer to a proximal side than the ultrasound element 21. At this time, as shown in Figs. 6 and 7, the annular expansion portion 12 is positioned radially outward of the ultrasound element 21. Therefore, in addition to the ultrasound reflected from the linear portion 11, the ultrasound element 21 can also receive the ultrasound reflected from the annular expansion portion 12. As described above, by making the annular expansion portion 12 movable to the same position as the ultrasound element 21 in the axial direction of the ultrasound element 21, the acquisition of positional information of the electrodes 30 by the ultrasound element 21 can be easily performed.

As shown in Figs. 6 and 7, in the medical device 1 according to the third modification example, a turned-back portion 18 of the linear portion 11 that is turned back via the second bent portion 17 is positioned radially outward of the ultrasound element 21. Therefore, the ultrasound element 21 can also receive the ultrasound reflected from the turned-back portion 18. The turned-back portion 18 is always disposed between the electrode 30a on the most proximal side of the electrodes 30 and the electrode 30j on the most distal side of the electrodes 30. Therefore, by acquiring the positional information of the turned-back portion 18 by the ultrasound element 21, a position of each electrode 30a to 30j can be specified with higher accuracy.

Furthermore, as shown in Fig. 7, in the medical device 1 according to the third modification example, a gap G, which is a gap between the proximal end and the distal end of the annular expansion portion 12, is positioned radially outward of the ultrasound element 21. The ultrasound transmitted and received by the ultrasound element 21 have a certain spread along the central axis O. Therefore, the ultrasound element 21 can specify the position of the gap G in which the reflection of the ultrasound from the annular expansion portion 12 is not detected. Similar to the turned-back portion 18, the gap G is always disposed between the electrode 30a on the most proximal side of the electrodes 30 and the electrode 30j on the most distal side of the electrodes 30. Therefore, by specifying the gap G by the ultrasound element 21, the positions of each electrode 30a to 30j can be specified with higher accuracy.

Fig. 8 is a view showing a fourth modification example of the medical device 1. As shown in Fig. 8, the distal end of the far side of the second lumen 42 may be positioned closer to a proximal side than the distal end of the far side of the first lumen 41. In other words, the far side communication hole 44 of the second lumen 42 may be positioned closer to a proximal side than the opening 46 of the first lumen 41. With such a configuration, the annular expansion portion 12 is not restricted in position on the distal side of the ultrasound element 21. Thereby, the annular expansion portion 12 can move to the same position as the ultrasound element 21 in the axial direction of the ultrasound element 21, and can further move closer to a proximal side than the ultrasound element 21. At this time, the annular expansion portion 12 is positioned radially outward of the ultrasound element 21. Therefore, the ultrasound element 21 can also receive the ultrasound reflected from the annular expansion portion 12. As described above, by making the annular expansion portion 12 movable to the same position as the ultrasound element 21 in the axial direction of the ultrasound element 21, the acquisition of positional information of the electrodes 30 by the ultrasound element 21 can be easily performed.

Fig. 9 is a schematic view of a medical system 100 according to the embodiment of the present disclosure. As shown in Fig. 9, the medical system 100 includes the medical device 1 and the ultrasound diagnostic apparatus 2. The ultrasound diagnostic apparatus 2 includes a drive unit 50, a display unit 51, an input unit 52, a storage unit 53, and a control unit 54.

The drive unit 50 incorporates a motor and rotationally drives the ultrasound element 21 via the shaft 22 of the medical device 1. The drive unit 50 is electrically connected to the ultrasound element 21 via a signal line extending in the shaft 22, and transmission and reception signals of the ultrasound element 21 are sent to the control unit 54. Specifically, the ultrasound element 21 receives the ultrasound reflected from the guide wire 10 and the ultrasound reflected from the body-cavity around the catheter 40, acquires signals based on the received ultrasound, and transmits the acquired signals to the control unit 54.

The display unit 51 displays and outputs a display screen generated by the control unit 54. The display unit 51 includes a display device such as a liquid crystal display or an organic EL display, for example.

The input unit 52 receives an input operation by an operator, and outputs the received input information to the control unit 54. The input unit 52 includes an input device such as a keyboard and a mouse, for example.

The storage unit 53 stores various information and programs for causing the control unit 54 to execute a specific function. The storage unit 53 may store an image generated by the control unit 54. The storage unit 53 includes a storage device such as a RAM and a ROM, for example.

The control unit 54 controls an operation of each configuration unit constituting the ultrasound diagnostic apparatus 2. The control unit 54 executes a specific function by reading a specific program. Specifically, the control unit 54 generates an image that can specify positions of the electrodes 30 with respect to the ultrasound element 21 in the circumferential direction based on the signals received from the ultrasound element 21 and causes the display unit 51 to display the image. The control unit 54 includes a processor or the like, for example.

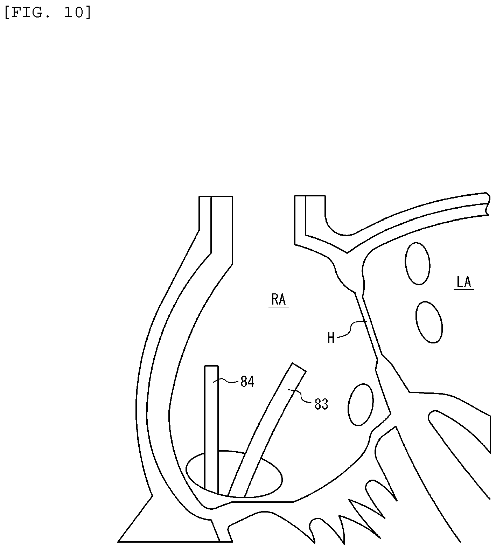

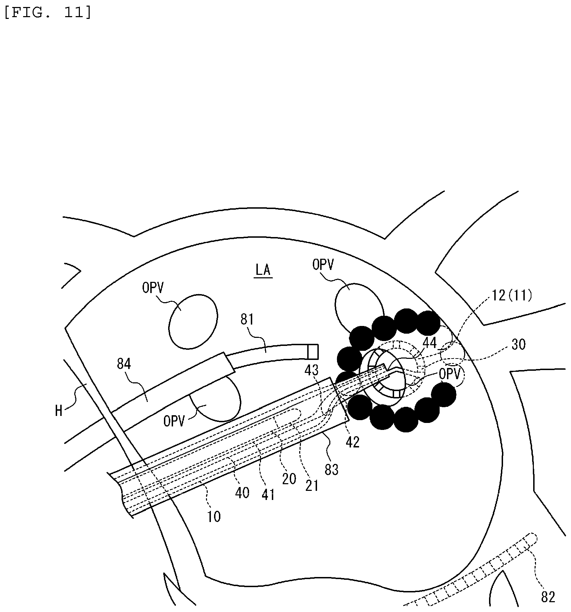

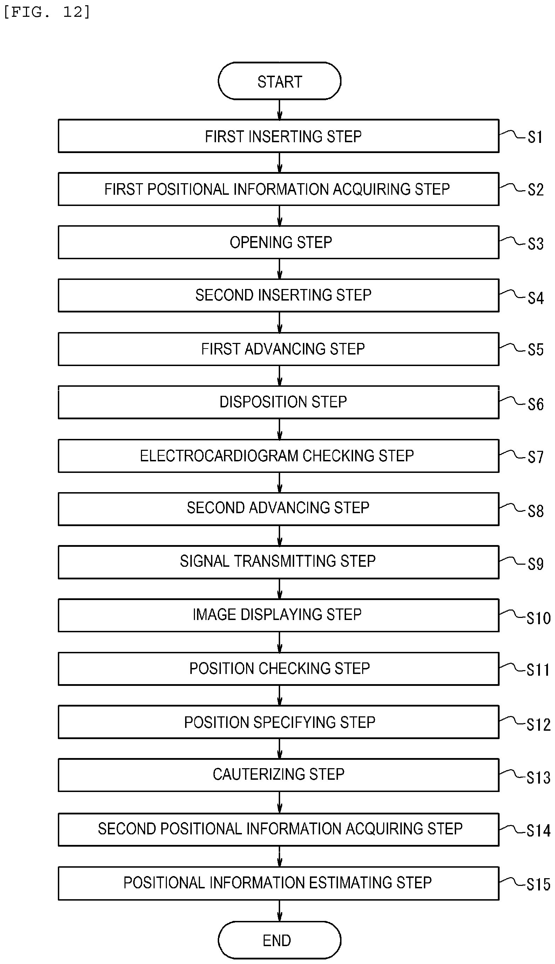

Hereinafter, a method for inserting the medical device 1 according to the embodiment of the present disclosure into a heart, and a method for observing the inside of the heart using the medical device 1, will be described. Fig. 10 is a view showing a right atrium. Fig. 11 is a view showing a left atrium. Fig. 12 is a flowchart showing each step of a method for inserting the medical device 1 into the heart and a method for observing the inside of the heart using the medical device 1.

First, as shown in Fig. 10, an operator such as a health care worker inserts a first sheath 83 and a second sheath 84 from a femoral vein into the right atrium RA (step S1: first inserting step). Thereafter, the operator penetrates the Brockenbrough needle inserted through the first sheath 83 or the second sheath 84 into a foramen ovale H separating the right atrium RA and the left atrium LA, and opens the left atrium LA from the right atrium RA (step S3: opening step). After step S1 and before step S3, in order to specify a position of the foramen ovale H, the medical device 1 (see Fig. 1 or the like) can be inserted through the first sheath 83, and surrounding positional information can be acquired using ultrasound of the first frequency band by the ultrasound element 21 (see Fig. 1 or the like) (step S2: first positional information acquiring step).

As shown in Fig. 11, when the right atrium RA and the left atrium LA are opened, the operator inserts the first sheath 83 and the second sheath 84 into the left atrium LA (step S4: second inserting step). Thereafter, the operator inserts the guide wire 10 (elongated body) inserted into the second lumen 42 into the first sheath 83 and advances the guide wire 10 to the left atrium LA (step S5: first advancing step). By the operator, the annular expansion portion 12 of the guide wire 10 is discharged from the far side communication hole 44 of the second lumen 42 and the far end of the first sheath 83, is expanded in an annular shape, and is disposed on an inner wall of a pulmonary vein ostium OPV as a body lumen (step S6: disposition step). Thereafter, the operator checks an electrocardiogram based on an electrical property of the inner wall of the pulmonary vein ostium OPV by the electrodes 30 disposed on the annular expansion portion 12 as needed (step S7: electrocardiogram checking step). By checking the electrocardiogram, among the plurality of electrodes 30a to 30j, it can be specified at which position corresponding to the electrode 30 the cauterization of the inner wall of the pulmonary vein ostium OPV is insufficient. At this time, a coronary sinus (CS) catheter 82 is indwelled in the coronary sinus, measures a difference in time of occurrence of an electric potential with the electrode 30, and is used for checking of electrical disconnection.

Next, as shown in Fig. 11, the operator advances the catheter 40 to the left atrium LA along the guide wire 10, inserts the ultrasound inspector 20 into the first lumen 41, and advances the ultrasound inspector 20 to the left atrium LA (step S8: second advancing step). In other words, the operator moves the ultrasound element 21 toward the electrodes 30 along the extending direction of the guide wire 10.

The ultrasound element 21 receives ultrasound reflected from the guide wire 10 and ultrasound reflected from the body-cavity around the catheter 40, acquires a signal based on the received ultrasound, and transmits the acquired signal to the ultrasound diagnostic apparatus 2 (step S9: signal transmitting step). The ultrasound diagnostic apparatus 2 displays an image based on the received signal on the display unit 51 (step S10: image displaying step) . The operator checks a position of the guide wire 10 based on the image based on the signal from the ultrasound element 21 displayed on the display unit 51 of the ultrasound diagnostic apparatus 2 (step S11: position checking step). Then, based on the checked position of the guide wire 10 and the electrocardiogram based on the electrical signal from the electrodes 30, the operator specifies a position of the electrode 30 corresponding to an insufficiently cauterized position on the inner wall of the pulmonary vein ostium OPV with respect to the ultrasound element 21 in the circumferential direction, based on the position of the guide wire 10 (step S12: position specifying step) .

Thereafter, as shown in Fig. 11, the operator advances the ablation catheter 81 as a cauterization device to the left atrium LA through the second sheath 84, and performs the ablation treatment. Specifically, the operator delivers the ablation catheter 81 to a position specified as a position where the cauterization is insufficient, and cauterizes the inner wall of the pulmonary vein ostium OPV (step S13: cauterizing step).

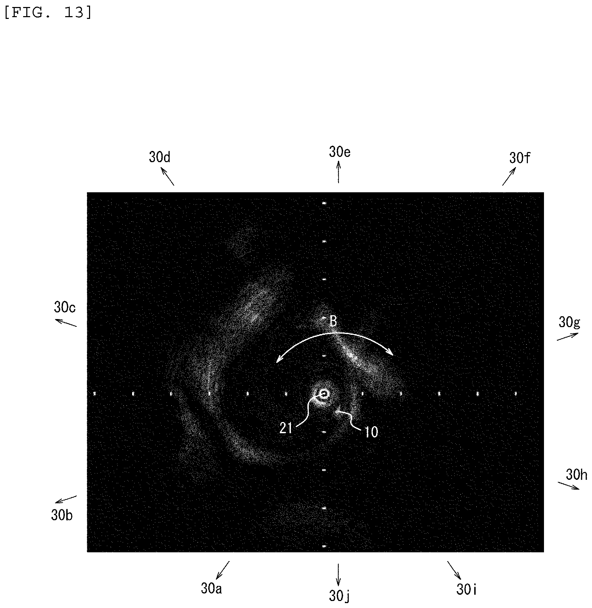

At the time of ablation treatment in step S13, in order to detect the position to be cauterized, the surrounding positional information can be acquired using the second frequency band by the ultrasound element 21 (step S14: second positional information acquiring step). Fig. 13 is a view showing an example of an image generated based on positional information acquired by the ultrasound element 21. As shown in Fig. 13, the positional information of the guide wire 10 is acquired by the ultrasound element 21. Therefore, the positional information of the electrodes 30a to 30j in the circumferential direction B can also be estimated (step S15: positional information estimating step).

Therefore, the electrical property obtained by the electrodes 30a to 30j can be obtained in association with the positional information obtained by the ultrasound element 21. As a result, while the cauterization at each point is sufficiently performed using the positional information obtained by the ultrasound element 21, the electrical property obtained by the electrodes 30a to 30j make it possible to detect that the electrical signals between specific sites are reliably disconnected.

Furthermore, according to the medical device 1 of the present embodiment, even when the cauterization at a specific position is insufficient, the positional relationship of the electrodes 30a to 30j in the circumferential direction with respect to the ultrasound element 21 can be easily specified. Therefore, the specific position where the cauterization is insufficient can be easily grasped, and for example, the cauterization at the specific position can be easily performed while checking the image shown in Fig. 13.

As described above, since the ultrasound element 21 can be moved along the extending direction of the guide wire 10 with respect to the guide wire 10, a desired positional information can be obtained by moving according to the cauterization site by the ablation catheter 81.

For example, when moving the catheter 40 along the extending direction of the guide wire 10 with respect to the guide wire 10 in each step of the above-mentioned step S8 (second advancing step) to step S15 (positional information estimating step), there is a possibility that the guide wire 10 may come out of the near side communication hole 43 of the second lumen 42 of the catheter 40 because the whole or a part of the first bent portion 14 of the guide wire 10 is accommodated inside the second lumen 42 of the catheter 40, and further the catheter 40 moves closer to a distal side than the distal end of the guide wire 10. In order to prevent the guide wire 10 from coming out of the near side communication hole 43 of the second lumen 42 of the catheter 40, the medical device 1 can be configured, for example, as follows.

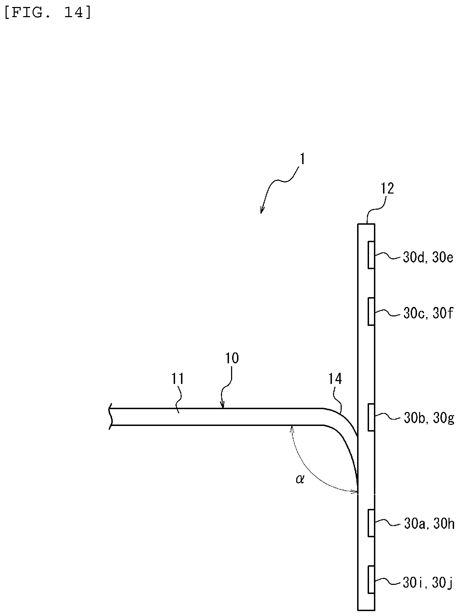

Fig. 14 is a view showing a part including the distal end of a guide wire 10 of the medical device 1. As shown in Fig. 14, it is preferable that the guide wire 10 be shape-memorized so that an angle α formed between the linear portion 11 and the annular expansion portion 12 via the first bent portion 14 is 135 ° or less. Thereby, the accommodation of the first bent portion 14 in the second lumen 42 can be suppressed.

The first bent portion 14 of the guide wire 10 and the distal portion 45 of the catheter 40 preferably have a certain degree of hardness. Specifically, for example, as shown in Fig. 11, when the catheter 40 is pushed to the far side or pulled into the near side with a load of 100 gf in a state where the far end of the annular expansion portion 12 is in contact with the tissue all around its circumference, it is preferable that, at least, the distal portion 45 of the catheter 40 has a hardness that does not follow the bending of the first bent portion 14, or the first bent portion 14 has a hardness that does not deform. In this case, it is more preferable that the distal portion 45 of the catheter 40 has a hardness that does not follow the bending of the first bent portion 14, and the first bent portion 14 has a hardness that does not deform. Thereby, the first bent portion 14 can be accommodated in the second lumen 42 and can be prevented from coming off.

Since an outer diameter of the core material forming the first bent portion 14 of the guide wire 10 is larger than an outer diameter of the core material forming the annular expansion portion 12 of the guide wire 10, it is preferable that the first bent portion 14 of the guide wire 10 is harder than the annular expansion portion 12 of the guide wire 10. Furthermore, the hardness of the first bent portion 14 of the guide wire 10 is preferably harder than the distal portion 45 of the catheter 40.

The distal portion 45 of the catheter 40 may be made of a harder member than the other parts of the catheter 40. The distal portion 45 of the catheter 40 may be configured to be harder than other parts, for example, by using a contrast marker 49 which is harder than the other parts.

The outer diameter of the first bent portion 14 of the guide wire 10 is preferably 55% or more and 80% or less with respect to the inner diameter of the second lumen 42. For example, when the outer diameter of the first bent portion 14 is 0.85 mm or more and 0.95 mm or less, the inner diameter of the second lumen 42 is preferably 1.25 mm or more and 1.45 mm or less. Thereby, even when the first bent portion 14 is accommodated in the second lumen 42, the first bent portion 14 can be smoothly discharged from the second lumen 42.

The inner surface of the second lumen 42 is preferably highly lubricious. From the viewpoint of enhancing the lubricity of the inner surface of the second lumen 42, it is preferable to use an olefin-based polymer such as polyethylene as the material of the catheter 40. Thereby, even when the first bent portion 14 is accommodated in the second lumen 42, the first bent portion 14 can be smoothly discharged from the second lumen 42.

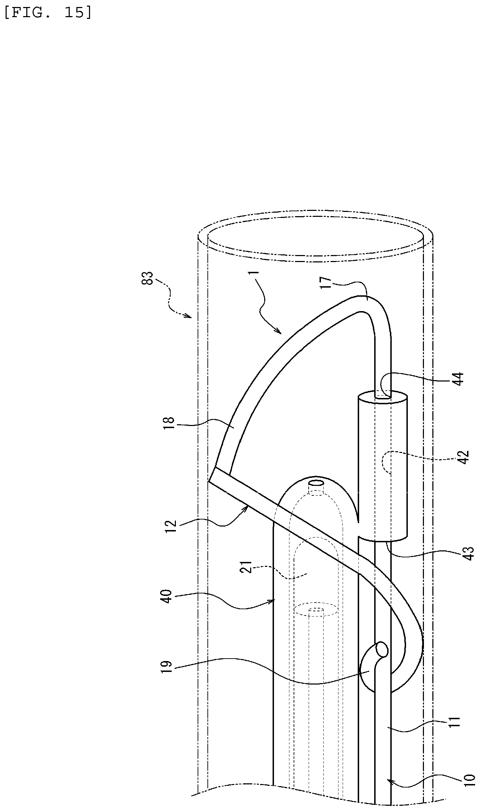

The case where the medical device 1 according to the third modification example shown in Figs. 6 and 7 is inserted into the heart will be described. Fig. 15 is a view showing a state in which the medical device 1 according to the third modification example is inserted into the first sheath 83. In Fig. 15, the first sheath 83 is shown as being transparent for the purpose of explanation.

As shown in Fig. 15, when inserting the medical device 1 according to the third modification example into the heart, the operator inserts the medical device 1 into the first sheath 83, in a state in which the second bent portion 17, the turned-back portion 18 and the annular expansion portion 12 of the guide wire 10 are discharged from the far side communication hole 44 of the second lumen 42, and are turned back to the proximal side at the second bent portion 17. At this time, as shown in Fig. 15, the annular expansion portion 12 is attached on the inner wall of the first sheath 83 and is not expanded in a complete annular manner. As shown in Fig. 15, a latch portion 19 may be formed at the distal end of the annular expansion portion 12, and the latch portion 19 may be engaged with the linear portion 11. The latch portion 19 can be formed, for example, by memorizing the distal end of the annular expansion portion 12 in a shape that can be engaged with the linear portion 11. By engaging the latch portion 19 with the linear portion 11 in this manner, the form of the medical device 1 inserted into the first sheath 83 can be maintained. The latch portion 19 may be positioned on the distal side of the far side communication hole 44. In that case, when the guide wire 10 and the catheter 40 are inserted into the heart, the guide wire 10 is first inserted into the heart. Thereafter, the catheter 40 can be moved along the guide wire 10. Thus, the annular expansion portion 12 can move to the same position as the ultrasound element 21 in the axial direction of the ultrasound element 21.

When observing the inside of the heart using the medical device 1, the operator moves the entire medical device 1 to the proximal side of the first sheath 83. Thereby, a part of the medical device 1 is discharged from the proximal end of the first sheath 83 with the second bent portion 17 at the head while maintaining the form. The annular expansion portion 12 expands to the outside of the linear portion 11 in an annular shape by being discharged from the first sheath 83. At this time, the engagement state of the latch portion 19 with the linear portion 11 may be released. In this way, the medical device 1 according to the third modification example can be deformed to the state shown in Figs. 6 and 7 to observe the inside of the heart.

The medical device, the medical system, the method for inserting the medical device, and the observation method using the medical device according to the present disclosure are not limited to the configurations of the embodiment described above, and it can be realized by various configurations without departing from the contents described in the claims.

### Industrial Applicability

The present disclosure relates to a medical device, a medical system, a method for inserting the medical device, and an observation method using the medical device.

### Reference Signs List

1 medical device
2 ultrasound diagnostic apparatus
10 guide wire (first elongated body)
11 linear portion
12 annular expansion portion
13 far end of an annular expansion portion
14 first bent portion
16 curved portion
17 second bent portion
18 turned-back portion
19 latch portion
20 ultrasound inspector
21 ultrasound element
22 shaft
23 tube
30, 30a∼30j electrodes
40 catheter (second elongated body)
41 first lumen
42 second lumen
43 near side communication hole
44 far side communication hole
45 distal portion
46 opening
49 contrast marker
50 drive unit
51 display unit
52 input unit
53 storage unit
54 control unit
81 ablation catheter (cauterization device)
82 CS catheter
83 first sheath
84 second sheath
100 medical system
A radial direction of a guide wire
B circumferential direction of a guide wire
O central axis of an ultrasound element
G gap
H foramen ovale
LA left atrium
OPV pulmonary vein ostium
RA right atrium
α angle between linear portion and annular expansion portion through first bent portion

## Claims

1. A medical device comprising:
a first elongated body that includes a linear portion and an annular expansion portion configured to expand in an annular shape;
an electrode that is fixed to the annular expansion portion of the first elongated body and is configured to detect an electrical property of an inner wall of a body lumen;
a second elongated body that includes a distal portion and a proximal portion, the second elongated body including a first lumen communicating from the distal portion to the proximal portion, and a second lumen parallel to the first lumen at the distal portion; and
an ultrasound element that is fixed to a distal portion of a shaft configured to rotate in a circumferential direction, is configured to move inside the first lumen of the second elongated body in an axial direction, and is configured to transmit and receive ultrasound,
wherein the annular expansion portion is configured to expand in an annular shape that is larger than an outer diameter of the second elongated body,
wherein the first elongated body is inserted into the second lumen, and the linear portion of the first elongated body is positioned in a direction orthogonal to the axial direction of the ultrasound element, and
wherein the ultrasound element receives ultrasound reflected from the first elongated body and ultrasound reflected from a body-cavity around the second elongated body.

2. The medical device according to Claim 1,
wherein a positional relationship of the ultrasound element with respect to the second elongated body in a circumferential direction of the second elongated body, is fixed.

3. The medical device according to Claim 1 or 2,
wherein a plurality of the electrodes are provided, and
wherein the plurality of the electrodes include two or more electrodes fixed at different positions along a circumferential direction of the first elongated body.

4. The medical device according to any one of Claims 1 to 3,
wherein the ultrasound element is disposed closer to a proximal side than the annular expansion portion of the first elongated body.

5. The medical device according to any one of Claims 1 to 3,
wherein the annular expansion portion is configured to move to the same position as the ultrasound element in the axial direction of the ultrasound element, and
wherein the ultrasound element receives ultrasound reflected from the annular expansion portion.

6. The medical device according to any one of Claims 1 to 5,
wherein a distal end of the second lumen is positioned closer to a distal side than a distal end of the first lumen.

7. The medical device according to any one of Claims 1 to 6,
wherein the second lumen extends along an extending direction of the first lumen.

8. The medical device according to any one of Claims 1 to 7,
wherein the first elongated body includes a curved portion that is curved so as to be separated radially outward of the ultrasound element with respect to a position of the ultrasound element.

9. A medical system comprising:
a medical device; and
an ultrasound diagnostic apparatus,
wherein the medical device includes
a first elongated body that includes a linear portion and an annular expansion portion configured to expand in an annular shape,
a plurality of electrodes that are fixed to the annular expansion portion of the first elongated body and is configured to detect an electrical property of an inner wall of a body lumen,
a second elongated body that includes a distal portion and a proximal portion, a first lumen communicating from the distal portion to the proximal portion, and a second lumen parallel to the first lumen at the distal portion, and
an ultrasound element that is fixed to a distal portion of a shaft configured to rotate in a circumferential direction, is configured to move inside the first lumen of the second elongated body in an axial direction, and transmits and receives ultrasound,
wherein the annular expansion portion is configured to expand in an annular shape that is larger than an outer diameter of the second elongated body,
wherein the first elongated body is inserted into the second lumen, and the linear portion of the first elongated body is positioned in a direction orthogonal to the axial direction of the ultrasound element,
wherein the ultrasound element receives ultrasound reflected from the first elongated body and ultrasound reflected from a body-cavity around the second elongated body, and transmits a signal that is based on the received ultrasound to the ultrasound diagnostic apparatus,
wherein the ultrasound diagnostic apparatus includes
a display unit and a control unit, and
wherein the control unit causes the display unit to display an image that is configured to specify a position of the electrode in a circumferential direction of the ultrasound element based on the signal from the ultrasound element.

10. A method for inserting a medical device that includes an elongated body having an electrode configured to detect an electrical property of an inner wall of a body lumen at a distal portion and an ultrasound element configured to move along an extending direction of the elongated body with respect to the elongated body, the method comprising:
a step of disposing the distal portion of the elongated body on the inner wall of the body lumen; and
a step of moving the ultrasound element toward the electrode along the extending direction of the elongated body.

11. The method for inserting the medical device according to Claim 10, further comprising:
a step of checking an electrocardiogram based on the electrical property of the inner wall of the body lumen by the electrode;
a step of checking a position of the elongated body based on an image that is based on a signal from the ultrasound element displayed on a display unit of an ultrasound diagnostic apparatus;
a step of specifying a position of an electrode corresponding to an insufficiently cauterized position in the inner wall of the body lumen in a circumferential direction of the ultrasound element, based on the position of the elongated body and the electrocardiogram; and
a step of delivering a cauterization device to the position of the specified electrode and cauterizing the inner wall of the body lumen.

12. An observation method using a medical device that includes an ultrasound element, comprising:
a step of acquiring positional information of surroundings using ultrasound in a first frequency band by the ultrasound element when opening a left atrium from a right atrium; and
a step of acquiring positional information of surroundings using ultrasound in a second frequency band by the ultrasound element when performing an ablation treatment, the second frequency band being a higher frequency band than the first frequency band.
